# EUROPÄISCHE PATENTSCHRIFT

(11) **EP 2 987 505 B1**
(45) Veröffentlichungstag und Bekanntmachung des Hinweises auf die Patenterteilung: **01.03.2017**
(21) Anmeldenummer: 15176858.7
(22) Anmeldetag: 15.07.2015
(51) Int. Cl.: A61L 2/24, A47L 15/42, A47L 15/00

(54) **SPÜLAUTOMAT, INSBESONDERE DESINFEKTIONSAUTOMAT, ENDOSKOPSPÜLER**
WASHING MACHINE, IN PARTICULAR DISINFECTION MACHINE, ENDOSCOPE WASHER
AUTOMATE DE RINÇAGE, NOTAMMENT AUTOMATE DE DESINFECTION, DISPOSITIF DE RINÇAGE D'ENDOSCOPE

(30) Priorität: 18.08.2014 DE 102014111718
(43) Veröffentlichungstag der Anmeldung: 24.02.2016
(73) Patentinhaber: Miele & Cie. KG, 33332 Gütersloh (DE)
(72) Erfinder: Heitmann, Michael, 33739 Bielefeld (DE)

(56) Entgegenhaltungen:
- EP-A1- 2 604 296
- EP-A2- 0 859 197
- DE-A1- 19 514 303
- JP-A- 2012 050 580

## Beschreibung

Spülautomat, insbesondere Desinfektionsautomat, Endoskopspüler und/oder dgl.

Die Erfindung betrifft einen Spülautomaten, insbesondere einen Desinfektionsautomaten, Endoskopspüler, mit einem Spülbehälter, der einen Spülraum mit einer mittels einer Hubtür fluiddicht verschließbaren Spülraumöffnung aufweist, und mit einer Antriebseinrichtung für ein motorisches Verfahren der Hubtür, wobei die Antriebseinrichtung ein Last tragendes Zugmittel aufweist.

Ein solcher Spülautomat ist aus der EP 2 604 296 A1 und aus der DE 19514303 A1 bekannt.

Der vorbekannte Spülautomat verfügt über einen Spülbehälter, der seinerseits einen Spülraum zur Aufnahme von zu reinigendem und/oder zu desinfizierendem Spülgut bereitstellt. Zwecks Beschickung des Spülraums mit zu reinigendem und/oder zu desinfizierendem Spülgut verfügt der Spülraum über eine Spülraumöffnung. Diese ist mittels einer Hubtür fluiddicht verschließbar.

Die Hubtür ist in Höhenrichtung des Spülautomaten verfahrbar. Bevorzugterweise erfolgt ein Verfahren der Hubtür motorisch, zu welchem Zweck eine Antriebseinrichtung vorgesehen ist. Diese verfügt über ein Last tragendes Zugmittel, das an die Hubtür angelenkt ist. Bei der aus der EP 2 604 296 A1 vorbekannten Konstruktion kommt als Zugmittel ein Zahnriemen zum Einsatz.

Aus Gründen der erhöhten Bediensicherheit verfügt der Spülautomat gemäß der EP 2 604 296 A1 über eine mechanische Verrastung, die eingreift, wenn es zu einem Ausfall des Zugmittels beispielsweise durch Reißen kommt. Der Spülautomat verfügt zu diesem Zweck über eine Arretiereinrichtung, die einen Arretierhebel einerseits und ein mit Aussparungen ausgerüstetes Widerlager andererseits aufweist. Kommt es zu einem Riss des Zugmittels, so schwenkt der unter Federvorspannung stehende Arretierhebel der Arretiereinrichtung aus und greift in eine Aussparung des Widerlagers ein, infolge dessen es zu einer mechanischen Verrastung der Hubtür kommt. Im Aktivierungsfall wird mittels der Arretiereinrichtung mithin eine Lagesicherung der Hubtür bewirkt, womit ein unkontrolliertes Herunterfahren der Hubtür sicher unterbunden ist.

Obgleich sich die Arretiereinrichtung des vorbekannten Spülautomaten im alltäglichen Praxiseinsatz bewährt hat, ist sie nicht frei von Nachteilen. So bedingt der konstruktive Aufbau der mechanischen Verrastung, dass die Bremswirkung erst beim Überfahren einer dafür vorgesehenen Aussparung im Widerlager einsetzt. Die Bremswirkung erfolgt zudem im Falle des Einrastens schlagartig, was zu einem hohen Krafteintrag ins Widerlager führt und zudem mit einer entsprechenden Geräuschentwicklung einhergeht.

Zur Beseitigung dieser dem Stand der Technik anhaftenden Nachteile ist es die Aufgabe der Erfindung, einen Spülautomaten bereitzustellen, der hinsichtlich seiner Sicherheitsmaßnahmen im Falle einer abstürzenden Hubtür weiterentwickelt ist.

Zur Lösung dieser Aufgabe wird mit der Erfindung ein Spülautomat dmit den Merkmalen von Anspruch 1 vorgeschlagen.

Das Last tragende Zugmittel, beispielsweise ein Zahnriemen oder ein Seil ist im Unterschied zum Stand der Technik nicht direkt, sondern unter Zwischenordnung einer Hebelanordnung an der Hubtür angeordnet. Die Hebelanordnung weist ein Klemmmittel auf, das parallel zu einer Bewegungsrichtung der Hubtür geführt ist. Die Hubtür und das Klemmmittel stehen mittels einer entsprechenden Zugfeder unter Federvorspannung und stützen sich an einem gemeinsamen Widerlager ab. Dabei sorgt diese Federvorspannung im Absturzfall der Hubtür dafür, dass sich die Hubtür und das Klemmmittel reibschlüssig an das Widerlager anlegen, womit ein Festsetzen der Hubtür erreicht ist. Damit bewirkt die erfindungsgemäße Ausgestaltung im Unterschied zum Stand der Technik keine formschlüssige, sondern eine reibschlüssige Abbremsung und Festsetzung der Hubtür. Diese reibschlüssige Abbremsung greift im Unterschied zum Stand der Technik unmittelbar und nicht erst nach Überfahren einer Raststelle. Die Abbremsung ist damit sanfter und materialschonender.

Die erfindungsgemäße Konstruktion bewirkt zudem, dass sich die Hubtür bei einem Verfahren zunächst von der zugehörigen Dichtung der Spülraumöffnung abhebt, so dass Beschädigungen der Dichtung infolge eines Anhaftens der Hubtür vermieden sind.

Wird die Spülraumtür im bestimmungsgemäßen Anwendungsfall angehoben, so drückt die Gewichtskraft der Hubtür die die Federvorspannung bewirkende Zugfeder zusammen. Dabei führt die Hebelanordnung eine Schwenkbewegung aus und hebt die Spülraumtür von der zugehörigen Dichtung der Spülraumtüröffnung ab. Die Hubtür lässt sich mithin frei bewegen und in Höhenrichtung verfahren. Wird nun der Kraftschluss unterbrochen, so drückt die hierdurch frei werdende Federkraft der Zugfeder die Hubtür gegen die Dichtung. Infolge der hierdurch einsetzenden Reibung zwischen Hubtür und Dichtung wird die Federkraft und damit das Anpressen der Hubtür verstärkt, so dass wiederum die Reibkraft ansteigt und letztendlich größer als die Gewichtskraft wird, so dass es zu einem Festsetzen der Hubtür kommt.

Es ist gemäß einem weiteren Merkmal der Erfindung vorgesehen, dass das Klemmmittel eine Klemmleiste ist. Diese ist in einer vertikal verlaufenden Führung angeordnet. Im bestimmungsgemäßen Verwendungsfall wird die Klemmleiste mithin in Höhenrichtung des Spülautomaten geführt. Die Klemmleiste ist in ihrer Längenausgestaltung so gewählt, dass im Betriebsfall ein sicheres Andrücken der Klemmleiste und der Hubtür an das Widerlager erfolgt, so dass unter Ausbildung des dann anliegenden Reibschlusses ein Abbremsen und Festsetzen der Hubtür erfolgen kann.

Das Zugmittel ist gemäß einem weiteren Merkmal der Erfindung an eine parallel zum Klemmmittel geführte Zugstange angeschlossen. Die Zugstange selbst ist an die Hubtür angeflanscht, so dass im bestimmungsgemäßen Verwendungsfall eine Krafteinleitung unter Zwischenschaltung der Zugstange in die Hubtür erfolgt.

Es ist gemäß einem weiteren Merkmal der Erfindung vorgesehen, dass die Hebelanordnung einen Schwenkhebel aufweist, der einendseitig verschwenkbar an der Zugstange und anderendseitig verschwenkbar an dem Klemmmittel angeordnet ist. Auf diese Weise wird im bestimmungsgemäßen Verwendungsfall eine Parallelverschiebung von Zugstange und Klemmmittel erreicht, die eine gewisse Ausrückbewegung mit sich bringt, was in vorteilhafter Weise zur Folge hat, dass die Hubtür von der Spülraumöffnungsdichtung abgehoben wird. Unnötige mechanische Belastungen der Dichtung können damit vermieden werden, was die Lebensdauer der Dichtung erhöht.

Vorzugsweise ist ein Anschlag vorhanden, durch den das Verschwenken des Schwenkhebels begrenzt ist. Dieser Anschlag verhindert zum einen, dass die Hubtür eine zu große Bewegung nach außen macht, also zu weit von der Spülraumöffnungsdichtung abgehoben wird, und damit beim anschließenden Öffnen mit Verkleidungsteilen kollidiert. Weiter wird vermieden, dass der Klemmhebel in eine senkrechte Strecklage schwenkt, aus der er beim anschließenden Verschließen der Hubtür nicht mehr zurückschwenken würde. Außerdem wird die das Federelement, also etwa die Zugfeder, vor Überdehnung geschützt. Der Anschlag ist vorteilhafterweise am Klemmmittel, insbesondere der Klemmleiste angeordnet.

Der Schwenkhebel ist gemäß einem weiteren Merkmal der Erfindung verdrehbar an der Hubtür angeordnet. Über den Schwenkhebel findet mithin ausgehend von der Zugstange eine Krafteinleitung in die Hubtür statt.

Gemäß einem weiteren Merkmal der Erfindung ist vorgesehen, dass die Hubtür einen Türrahmen aufweist, der innenseitig den Schwenkhebel verdrehbar trägt. Eine kompakte und in Frontansicht auf den Spülautomaten benutzerseitig nicht einsehbare Anordnung des Schwenkhebels ist so erreicht.

Je Türrahmenseite sind gemäß einem weiteren Merkmal der Erfindung vier Schwenkhebel vorgesehen. Damit ist sichergestellt, dass auch bei sich in Offenstellung befindlicher Hubtür noch zumindest zwei Schwenkhebel für ein bestimmungsgemäßes Anpressen der Klemmleiste und der Hubtür an das zugehörige Widerlager sorgen.

Die erfindungsgemäße Ausgestaltung sieht im Unterschied zum Stand der Technik ein Klemmmittel vorzugsweise in der Ausgestaltung als Klemmleiste vor, das in Wechselwirkung mit der Hubtür eine reibschlüssige Selbsthemmung bereitstellt. Diese greift verhältnismäßig geräuschlos ein und ermöglicht zudem eine stufenlose Lagefixierung der Hubtür. Dabei erfolgt die reibschlüssige Selbsthemmung nach der Erfindung dadurch, dass eine parallel zur Hubtür geführte Klemmleiste vorgesehen ist. Diese wird federbelastet von der Hubtür weggedrückt bzw. im Versagensfall zueinander geführt, was in Wechselwirkung mit dem dafür vorgesehenen Widerlager zur Selbsthemmung und damit zur Notabbremsung führt. Dabei greift die Notbremsung in vorteilhafter Weise unabhängig vom Versagensfall immer dann, wenn die mit der Klemmleiste gekoppelte Zugstange kraftlos gestellt wird. Insofern greift die erfindungsgemäße Selbsthemmung nicht nur im Falle des Reißens des Zugmittels, beispielsweise des Seils, sondern auch im Falle eines defekten Antriebs, eines defekten Getriebes und/oder dgl.

Weitere Merkmale und Vorteile der Erfindung ergeben sich aus der nachfolgenden Beschreibung anhand der Figuren. Dabei zeigen
- Fig. 1: ausschnittsweise in einer schematischen Seitenansicht den erfindungsgemäßen Spülautomaten;
- Fig. 2: den erfindungsgemäßen Spülautomaten nach Fig. 1 ausschnittsweise in einer Draufsicht von oben und
- Fig. 3: in einer schematischen Darstellung das Wirkprinzip nach der erfindungsgemäßen Ausgestaltung.

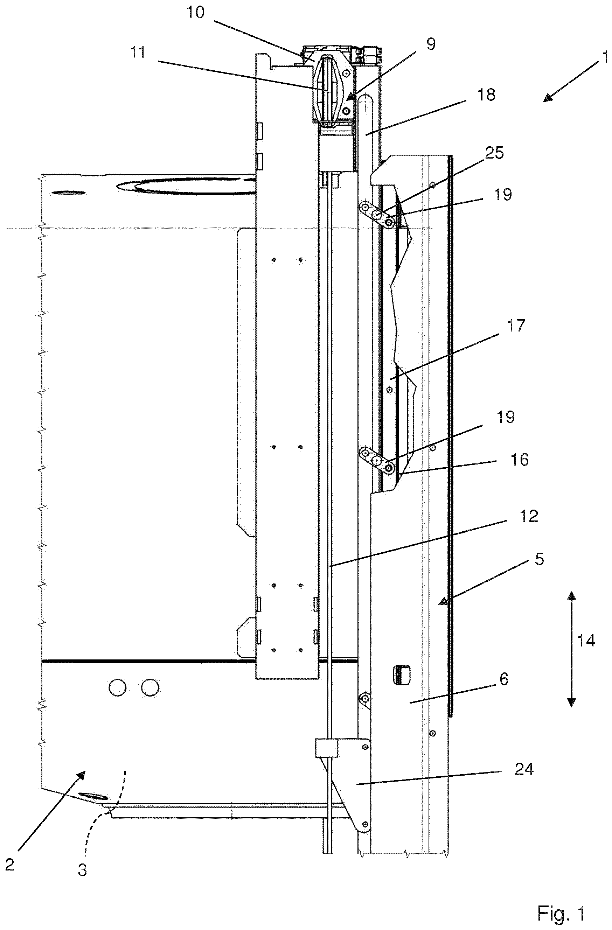

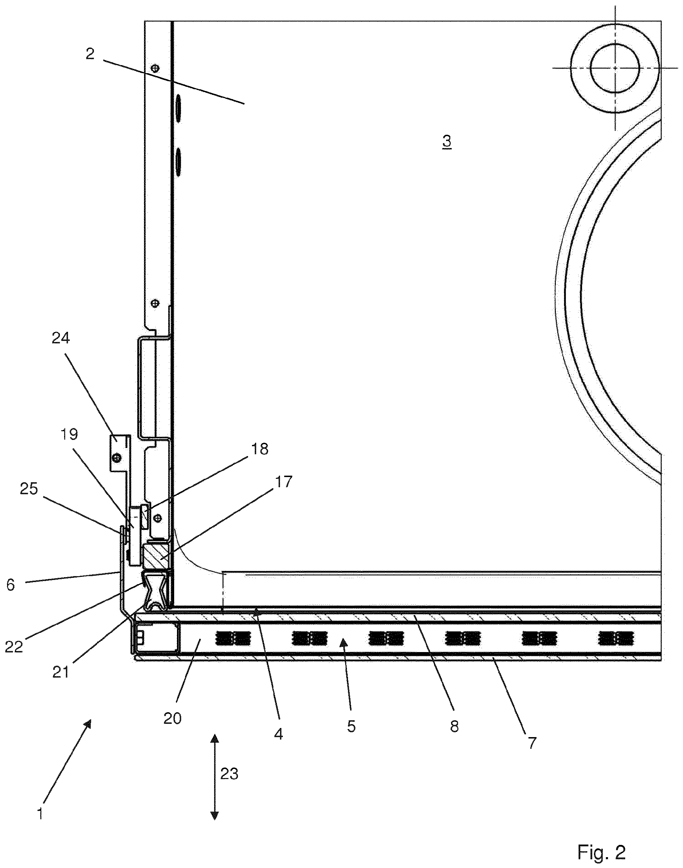

Die Figuren 1 und 2 lassen ausschnittsweise den erfindungsgemäßen Spülautomaten 1 erkennen, und zwar aus einer Seitenansicht gemäß Fig. 1 und einer Draufsicht von oben gemäß Fig. 2.

Wie sich aus einer Zusammenschau der Figuren 1 und 2 ergibt, verfügt der Spülautomat in an sich bekannter Weise über einen Spülbehälter 2, der einen Spülraum 3 bereitstellt. Der Spülraum 3 ist mittels einer Spülraumöffnung 4 zugänglich, wobei die Spülraumöffnung 4 mittels einer Hubtür 5 fluiddicht verschließbar ist. Dabei ist die Hubtür 5 in Bewegungsrichtung 14, d.h. in Höhenrichtung des Spülautomaten 1 verfahrbar ausgebildet.

Die Hubtür 5 stellt eine Außenscheibe 7 und eine Innenscheibe 8 bereit, die unter Zwischenordnung eines Profilrahmens 20 beabstandet voneinander angeordnet sind. Zur Anordnung der Hubtür 5 am Spülbehälter 2 dient ein Türrahmen 6.

Die Hubtür 5 ist motorisch verfahrbar ausgebildet, zu welchem Zweck eine Antriebseinrichtung 9 vorgesehen ist, die im gezeigten Ausführungsbeispiel über einen pneumatischen Antrieb 10 und eine Umlenkrolle 11 verfügt, über die ausgehend vom pneumatischen Antrieb 10 ein Zugmittel 12 in Form eines Seils geführt ist.

Das Zugmittel 12 ist mit seinem antriebsentfernten Endabschnitt über eine Anlenkung 24 an eine Zugstange 18 angeschlossen. Diese steht wiederum über Schwenkhebel 19 mit der Hubtür 5 in Verbindung, wobei die Schwenkhebel 19 jeweils um die Schwenkachse 25 verdrehbar innenseitig des Türrahmens 6 der Hubtür 5 angeordnet sind, wie dies insbesondere die Darstellung nach Fig. 2 zeigt.

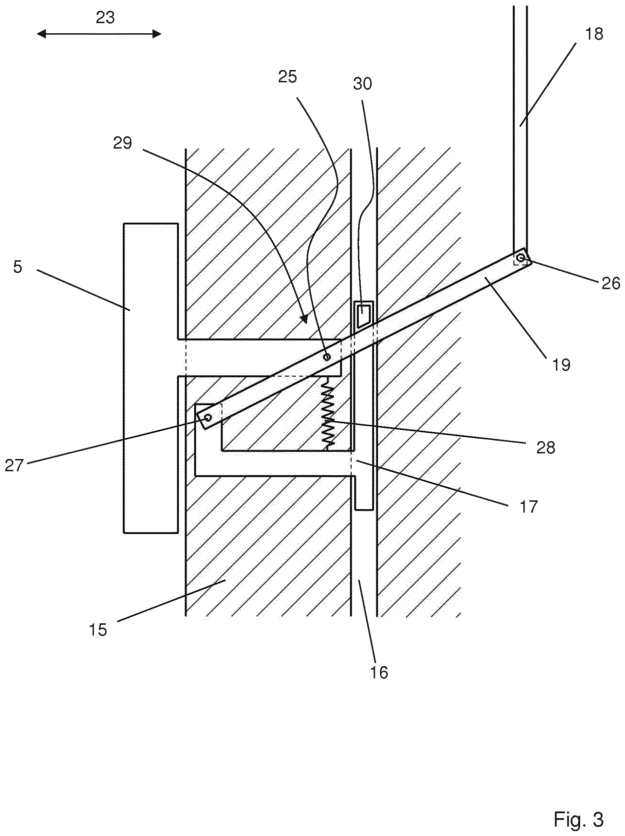

Die Schwenkhebel 19 sind jeweils einendseitig um die Drehachse 26 verschwenkbar an der Zugstange 18 sowie anderendseitig um die Drehachse 27 verschwenkbar an einem Klemmmittel 17 in Form einer Klemmleiste angeordnet. Dabei ist das Klemmmittel 17 in Bewegungsrichtung 14 innerhalb einer Führung 16 gelagert. Vorzugsweise ist ein Anschlag 30 vorhanden, durch den das Verschwenken des Schwenkhebels 19 begrenzt ist; der Anschlag 30 ist dabei insbesondere am Klemmmittel 17 angeordnet. Das Klemmmittel 17 und die Hubtür 5 stehen unter Zwischenordnung von Zugfedern 28 unter Federvorspannung, wie sich dies insbesondere aus der Prinzipdarstellung nach Fig. 3 ergibt. Dabei stützen sich die Hubtür 5 und das Klemmmittel 17 gegenüber dem gemeinsamen Widerlager 15 ab, wie sich dies ebenfalls aus der Darstellung nach Fig. 3 ergibt.

Die Funktionsweise der erfindungsgemäßen Ausgestaltung ergibt sich insbesondere aus der Wirkprinzipdarstellung nach Fig. 3.

Soll im bestimmungsgemäßen Verwendungsfall ein Anheben der Hubtür 5 erfolgen, so wird über den pneumatischen Antrieb 10 eine Zugkraft in das als Zugmittel 12 dienende Seil eingeleitet, infolge dessen eine Krafteinleitung in die Zugstange 18 stattfindet. Infolge dieser Krafteinleitung verdrehen die Schwenkhebel 19 und die Hubtür 5 wird infolge des Anliegens des Klemmmittels 17 an der Begrenzung der Führung 16 von der Dichtung 21 bzw. dem Halter 22 für die Dichtung 21 in Bewegungsrichtung 23 abgedrückt. Über die Schwenkhebel 19 findet des Weiteren eine Krafteinleitung in die Hubtür 5 statt, so dass diese in Höhenrichtung nach oben verfährt. Infolge der durch die Schwenkhebel 19 eingeleiteten Verfahrbewegung des Klemmmittels 17 werden die zwischen Hubtür 5 und Klemmmittel 17 angeordneten Federn 28 gespannt.

Kommt es nun zu einem Ausfall der Antriebseinrichtung 9, sei es, dass der Antrieb 10 ausfällt, die Umlenkrolle 11 bricht, das Zugmittel 12 reißt oder ein ähnlicher Schaden auftritt, der zur Kraftlosstellung der Hebelanordnung 29 führt, so werden die Hubtür 5 und das Klemmmittel 17 aufgrund der auf sie einwirkenden Federkraft in Bewegungsrichtung 23 aufeinander zugeführt und legen sich an das gemeinsame Widerlager 15 an. Infolge dessen kommt es zu einem Reibschluss, der zur Abbremsung der Hubtür 5 und zu einem Festsetzen derselben führt. Ein Abstürzen der Hubtür 5 auch bei defekter Antriebseinrichtung 9 ist damit sicher unterbunden.

Das Klemmmittel 17 ist innerhalb der Führung 16 beidseitig geführt, d.h. das Klemmmittel 17 überträgt Horizontalkräfte in beide Richtungen, womit es die Hubtür 5 auf die Dichtung 21 pressen und im umgekehrten Fall die Hubtür von der Dichtung 22 abheben kann. Die Zugfedern 28 sind bevorzugter Weise so ausgelegt, dass sie beim Anheben durch das Gewicht der Hubtür 5 zusammengedrückt werden und andererseits bei Ausfall beispielsweise des Zugmittels 12 die Hubtür 5 unmittelbar gegen die Dichtung 21 drücken, was dann zur Selbsthemmung führt. Idealerweise liegt die durch die Zugfedern 28 induzierte Federkraft nur knapp unterhalb der Gewichtskraft der Hubtür 5.

Für die vorbeschriebene Selbsthemmung ist der Winkel zwischen Schwenkhebel 19 einerseits und der Reibflächennormalen kleiner als der Spitzwinkel im Dreieck aus Normalkraft, Reibkraft und resultierender Kraft.

Neben Zugfedern in vertikaler, horizontaler oder diagonaler Richtungen sind auch Druckfedern in unterschiedlichen Richtungen oder Schenkelfedern denkbar, die ein Drehmoment in die Schwenkhebel einleiten.

Ist die Dichtung 21 als aufblasbare Dichtung ausgebildet, ist für die Bauteilabmessungen ferner maßgeblich, dass die beim Aufblasen der Dichtung 21 auftretenden Horizontalkräfte die Hubtür 5 nicht wegdrücken können. Auch hier ist ein möglichst spitzer Winkel zwischen Schwenkhebel 19 einerseits und der Flächennormalen andererseits zu wählen.

### Bezugszeichen

- 1: Spülautomat
- 2: Spülbehälter
- 3: Spülraum
- 4: Spülraumöffnung
- 5: Hubtür
- 6: Türrahmen
- 7: Außenscheibe
- 8: Innenscheibe
- 9: Antriebseinrichtung
- 10: pneumatischer Antrieb
- 11: Umlenkrolle
- 12: Zugmittel
- 13: Hebelanordnung
- 14: Bewegungsrichtung (Höhenrichtung)
- 15: Widerlager
- 16: Führung
- 17: Klemmmittel
- 18: Zugstange
- 19: Schwenkhebel
- 20: Profilrahmen
- 21: Dichtung
- 22: Halteleiste
- 23: Bewegungsrichtung (Vertikalrichtung)
- 24: Anlenkung
- 25: Schwenkachse
- 26: Drehachse
- 27: Drehachse
- 28: Zugfeder
- 29: Hebelanordnung
- 30: Anschlag

## Patentansprüche

1. Spülautomat, insbesondere Desinfektionsautomat oder Endoskopspüler, mit einem Spülbehälter (2), der einen Spülraum (3) mit einer mittels einer Hubtür (5) fluiddicht verschließbaren Spülraumöffnung (4) aufweist, und mit einer Antriebseinrichtung (9) für ein motorisches Verfahren der Hubtür (5), wobei die Antriebseinrichtung (9) ein Last tragendes Zugmittel (12) aufweist,
wobei zwischen dem Zugmittel (12) und der Hubtür (5) eine Hebelanordnung (29) angeordnet ist, die ein parallel zu einer Bewegungsrichtung (14) der Hubtür (5) geführtes Klemmmittel (17) aufweist,
**dadurch gekennzeichnet,**
**dass** sich die Hubtür (5) und das Klemmmittel (17) unter Federvorspannung gegenüber einem gemeinsamen Widerlager (15) abstützen,
**dass** das Zugmittel (12) an eine parallel zum Klemmmittel (17) geführte Zugstange (18) angeschlossen ist,
und die Hebelanordnung (29) einen Schwenkhebel (19) aufweist, der einendseitig verschwenkbar an der Zugstange (18) und anderendseitig verschwenkbar an dem Klemmmittel (17) angeordnet ist.

2. Spülautomat nach Anspruch 1,
**dadurch gekennzeichnet, dass**
das Klemmmittel (17) eine in einer vertikal verlaufenden Führung (16) angeordnete Klemmleiste ist.

3. Spülautomat nach Anspruch 1 oder 2,
**dadurch gekennzeichnet, dass**
der Schwenkhebel (19) verdrehbar an der Hubtür (5) angeordnet ist.

4. Spülautomat nach einem der vorhergehenden Ansprüche,
**dadurch gekennzeichnet, dass**
die Hubtür (5) einen Türrahmen (6) aufweist, der innenseitig den Schwenkhebel (19) verdrehbar trägt.

5. Spülautomat nach einem der vorhergehenden Ansprüche,
**dadurch gekennzeichnet, dass** das Verschwenken des Schwenkhebels (19) durch einen Anschlag (30) begrenzt ist.

6. Spülautomat nach einem der vorhergehenden Ansprüche,
**dadurch gekennzeichnet, dass**
je Türrahmenseite vier Schwenkhebel (19) vorgesehen sind.

7. Spülautomat nach einem der vorhergehenden Ansprüche,
**dadurch gekennzeichnet, dass**
zwischen dem Widerlager (15) und der Hubtür (5) eine Dichtung (21) angeordnet ist.

## Claims

1. Automatic rinsing machine, in particular a disinfection machine or endoscope rinsing machine, comprising a rinsing container (2) that has a rinsing chamber (3) comprising a rinsing chamber opening (4) that can be closed in a fluid-tight manner by means of a lift door (5), and comprising a drive device (9) for motorised movement of the lift door (5), the drive device (9) having a load-bearing tension means (12), a lever arrangement (29) being arranged between the tension means (12) and the lift door (5) and having a clamping means (17) that is guided in parallel with a direction of movement (14) of the lift door (5),
**characterised in that**
the lift door (5) and the clamping means (17) are supported under spring preload with respect to a common abutment (15),
**in that** the tension means (12) is connected to a tension rod (18) that is guided in parallel with the clamping means (17),
and **in that** the lever arrangement (29) comprises a swivelling lever (19) that at one end is arranged such that it can swivel on the tension rod (18) and at the other end is arranged such that it can swivel on the clamping means (17).

2. Automatic rinsing machine according to claim 1,
**characterised in that**
the clamping means (17) is a clamping strip arranged in a vertically extending guide (16).

3. Automatic rinsing machine according to either claim 1 or claim 2,
**characterised in that**
the swivelling lever (19) is arranged so as to be rotatable on the lift door (5).

4. Automatic rinsing machine according to any of the preceding claims,
**characterised in that**
the lift door (5) has a door frame (6) that supports the swivelling lever (19) on the inside such that it can rotate thereon.

5. Automatic rinsing machine according to any of the preceding claims,
**characterised in that**
the swivelling movement of the swivelling lever (19) is restricted by a stop (30).

6. Automatic rinsing machine according to any of the preceding claims,
**characterised in that**
four swivelling levers (19) are provided on each side of the door frame.

7. Automatic rinsing machine according to any of the preceding claims,
**characterised in that**
a seal (21) is arranged between the abutment (15) and the lift door (5).

## Revendications

1. Machine de lavage automatique, en particulier machine de désinfection automatique ou machine de rinçage d'endoscopes, avec une cuve de lavage (2), laquelle présente une chambre de rinçage (3) avec une ouverture de la chambre de rinçage (4) qui peut être fermée de manière étanche vis-à-vis des fluides, au moyen d'une porte à guillotine (5), et avec un mécanisme d'entraînement (9) pour un mouvement motorisé de la porte à guillotine (5), dans lequel le mécanisme d'entraînement (9) présente un moyen de traction (12) supportant une charge, dans laquelle une disposition à leviers (29) est disposée entre le moyen de traction (12) et la porte à guillotine (5), laquelle disposition à leviers présente un moyen de serrage (17) guidé parallèlement à une direction de mouvement (14) de la porte à guillotine (5),
**caractérisée en ce que**
la porte à guillotine (5) et le moyen de serrage (17) se soutiennent sous l'action d'une précontrainte élastique par rapport à un élément commun de contre-appui (15),
le moyen de traction (12) est raccordé à une tige de traction (18) guidée parallèlement au moyen de serrage (17),
et la disposition à leviers (29) présente un levier pivotant (19), lequel est disposé, à une extrémité, au niveau de la tige de traction (18) de manière à pouvoir pivoter et lequel est disposé, à l'autre extrémité, au niveau du moyen de serrage (17) de manière à pouvoir pivoter.

2. Machine de lavage automatique selon la revendication 1,
**caractérisée en ce que**
le moyen de serrage (17) est une barre de serrage disposée dans un guidage (16) se prolongeant à la verticale.

3. Machine de lavage automatique selon la revendication 1 ou 2,
**caractérisée en ce que**
le levier pivotant (19) est disposé au niveau de la porte à guillotine (5) de manière à pouvoir tourner.

4. Machine de lavage automatique selon l'une des revendications précédentes,
**caractérisée en ce que**
la porte à guillotine (5) présente un cadre de porte (6) qui supporte intérieurement, de manière à pouvoir tourner, le levier pivotant (19).

5. Machine de lavage automatique selon l'une des revendications précédentes,
**caractérisée en ce que**
le pivotement du levier pivotant (19) est limité par une butée (30).

6. Machine de lavage automatique selon l'une des revendications précédentes,
**caractérisée en ce que**
quatre leviers pivotants (19) sont prévus pour chaque côté du cadre de porte.

7. Machine de lavage automatique selon l'une des revendications précédentes,
**caractérisée en ce que**
une garniture étanche (21) est disposée entre l'élément de contre-appui (15) et la porte à guillotine (5).
